# EUROPÄISCHE PATENTSCHRIFT

(11) **EP 2 235 038 B1**
(45) Veröffentlichungstag und Bekanntmachung des Hinweises auf die Patenterteilung: **05.06.2019**
(21) Anmeldenummer: 09808930.3
(22) Anmeldetag: 30.12.2009
(51) Int. Cl.: C07K 1/04, C08G 69/10, C07K 1/02

(54) **Tetrapeptidkombination**
Tetrapeptide Combination
Combinaison de Tetrapeptides

(30) Priorität: 30.12.2008 DE 102008063256
(43) Veröffentlichungstag der Anmeldung: 06.10.2010
(73) Patentinhaber: GKL-Biotec AG, 8157 Dielsdorf (CH)
(72) Erfinder: KLETT-LOCH, Guenther, 68309 Mannheim (DE)
(74) Vertreter: Patent- und Rechtsanwälte Ullrich & Naumann
(86) Internationale Anmeldenummer: PCT/DE2009/001819
(87) Internationale Veröffentlichungsnummer: WO 2010/075849

(56) Entgegenhaltungen:
- EP-A1- 0 983 080
- DE-A1- 10 327 518
- JOHN M. OSTRESH, JAMES H. WINKLE, VINCE T. HAMASHIN, RICHARD A. HOUGHTEN: "Peptide Libraries: Determination of Relative Reaction Rates of Protected Amino Acids in Competitive Couplings" BIOPOLYMERS, Bd. 34, Nr. 12, Dezember 1994 (1994-12), Seiten 1681-1689, XP002578391
- CHRISTIAN BIRR: "THE GMP--BASED DRUG SUBSTANCE SCTL DEVELOPMENT AIMING AT PREVENTION OF OPPORTUNISTIC INFECTIONS AFTER X--RAY-- AND CHEMOTHERAPY OF CANCER.. A SYNTHETIC COMBINATORIAL TETRAPEPTIDE LIBRARY SUBSTITUTION FOR CALF THYMUS EXTRACT", BIOTECHNOLOGIA ACTA,, vol. 6, no. 4, 2013, pages 162-170, XP055099145,
- C. Birr, G. Braum, W. Hirt, G. H. Klett-Loch: "Statistic Combination f Thymus Peptides, a Synthetic Library Mimicing the Physiological Environment" In: S. Bajusz, F. Hudecz: "Peptides 1998. Proceedings of the 25th European Peptide Symposium", 1998 ISBN: 978-963-05-7622-2 pages 62-63,
- DOOLEY C T ET AL: "SELECTIVE LIGANDS FOR THE MU, DELTA AND KAPPA OPIOID RECEPTORS IDENTIFIED FROM A SINGLE MIXTURE BASED TETRAPEPTIDE POSITIONAL SCANNING COMBINATORIAL LIBRARY", JOURNAL OF BIOLOGICAL CHEMISTRY, AMERICAN SOCIETY FOR BIOCHEMISTRY AND MOLECULAR BIOLOGY, US, vol. 278, no. 30, 24 July 1998 (1998-07-24), pages 18848-18856, XP002925773, ISSN: 0021-9258, DOI: 10.1074/JBC.273.30.18848
- ARAKAWA T ET AL: "Biotechnology applications of amino acids in protein purification and formulations", AMINO ACIDS ; THE FORUM FOR AMINO ACID AND PROTEIN RESEARCH, SPRINGER-VERLAG, VI, vol. 33, no. 4, 16 March 2007 (2007-03-16) , pages 587-605, XP019565540, ISSN: 1438-2199, DOI: 10.1007/S00726-007-0506-3

## Beschreibung

Die vorliegende Erfindung betrifft eine Tetrapeptidkombination, ein Syntheseverfahren von Tetrapeptidkombinationen sowie eine Peptid-Bibliothek.

Aus der Europäischen Patentschrift 0 983 080 B1 sind bereits synthetische, statistische Thymuspeptid-Kombinationen und deren Verwendung als immunologisch und/oder endokrinologisch wirkendes Präparat bekannt. Das Thymuspeptid-Kombinationen enthaltende Präparat ist durch Aneinanderreihung von chemisch geeignet derivatisierten Aminosäuren zu kurzkettigen Peptiden erhältlich, wobei das für das Thymusgewebe charakteristische Mengenverhältnis und Muster der Aminosäuren ausgebildet wird, so dass mit dem Präparat eine dosisabhängige Steigerung der Proliferation von humanen Lymphozyten zu erzielen ist. Ausgangspunkt zur Herstellung der synthetischen, statistischen Thymuspeptid-Kombination ist die Analyse von einzelnen Peptiden aus Partialhydrolysaten von Thymusproteinen. Durch Reaktion von Aminosäuren in einem für das Thymusgewebe charakteristischen Mengenverhältnis und Muster sind die aus den Partialhydrolysaten bekannten Peptide aus synthetischen Aminosäuren synthetisierbar, die in ihrer Gesamtheit die charakteristische synthetische, statistische Thymuspeptid-Kombination bilden und so ein BSE-Risiko ausgeschlossen werden kann.

Auch aus der Deutschen Offenlegungsschrift 103 27 518 A1 ist ein synthetische Peptidkombinationen enthaltendes Präparat und ein Verfahren zu deren Herstellung bekannt. Bei der in dieser Anmeldung beanspruchten Erfindung handelt es sich um Partialhydrolysate aus menschlichen Gewebe, wobei das Ergebnis der Partialhydrolysate von speziellen Enzymkombinationen abhängig ist, dass heißt, das Ergebnis durch die Auswahl von Enzymen in speziellen Kombinationen gezielt steuerbar ist.

Die Veröffentlichung C. Birr, G. Braum, W. Hirt, G. H. Klett-Loch: "Statistic Combination of Thymus Peptides, a Synthetic Library Mimicing the Physiological Environment" In: W. Bajusz, F.Hudecz: "Peptides 1998. Proceedings of the 25th European Peptide Symposium", 1998, Seiten 62-63", beschreibt weitere Versuche zu der in der Europäischen Patentschrift 0 983 080 B1 beschriebenen synthetischen Peptidkombination GKL-01 im Vergleich zu durch Hydrolyse gewonnenen Peptidkombinationen.

Dooley C T et al, "Selective ligands for the mu, delta and kappa opioid receptors identified from a single mixture based tetrapeptide positional scanning combinatorial library", Journal of Biological Chemistry, American Society for Biochemistry and Molecular Biology, US, Bd. 278, Nr. 30, 24. Juli 1998 (1998-07-24), Seiten 18848-18856, beschreibt eine kombinatorische Bibliothek aus 6,250,000 Tetrapeptiden, die in Bindungsassays bezüglich der Bindung der drei Opioid Rezeptoren µ, δ und κ gemessen wurde. Bei dieser Peptid-Bibliothek handelt es sich um eine synthetische kombinatorische Bibliothek. Dabei wurden neue hochaktive die Rezeptoren bindende Peptide ermittelt.

Ausgehend von dem oben zitierten Stand der Technik, hat sich die Anmelderin der vorliegenden Erfindung die Aufgabe gestellt, Peptidkombinationen in gleichbleibender reproduzierbarer Qualität und Quantität zur Verfügung zu stellen, die beispielsweise Grundlage eines Präparates für ein Therapieverfahren sind.

Darüber hinaus ist es Aufgabe der vorliegenden Erfindung, ein Syntheseverfahren zur Herstellung von Peptidkombinationen zur Verfügung zu stellen, mit dem die Peptidkombinationen in reproduzierbarer Weise bezüglich ihrer Qualität und Quantität herstellbar sind.

Die erfindungsgemäße Tetrapeptidkombination ist herstellbar aus einer Mischung (A), enthaltend
- eine Anzahl x an Aminosäuren mit geschützter Säuregruppe oder eine Anzahl z an Peptiden mit mittels Schutzgruppe geschützter Säuregruppe und aktivierter Aminogruppe, wobei die Aminosäuren in der Mischung (A) in bestimmten einstellbaren Molverhältnissen vorliegen, und wobei x = 11 ist, und eine Mischung (B), enthaltend
- eine Anzahl y an Aminosäuren mit mittels Schutzgruppe geschützter Aminogruppe, wobei die Aminosäure-Molverhältnisse der Mischung (B) gleich den Aminosäure-Molverhältnisse der Mischung (A) sind, und wobei die Anzahl x = y ist, wobei den Peptiden eine Mischung aus natürlichen Aminosäuren in einem Verhältnis von 100 g +/- 25 g Peptide : 80 g +/- 20 g Aminosäuremischung zugesetzt ist.

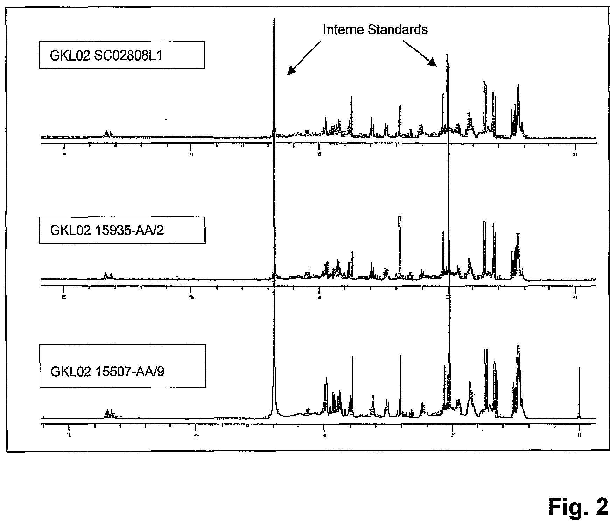

Erfindungsgemäß ist erkannt worden, dass durch das Zusammenführen von zwei Aminosäure-Mischungen A und B, die eine identische Anzahl Aminosäuren enthalten, die bezüglich ihrer Molverhältnisse einstellbar sind, dass heißt zwei in Ihrer Aminosäurezusammensetzung, ausgenommen Ihrer Schutzgruppen, identische Mischungen aus Aminosäuren miteinander in Reaktion zu bringen, bzw. weiterführend synthetisierte Peptide aus den Mischungen A und B mit mittels Schutzgruppe geschützter Säuregruppe und aktivierter Aminogruppe weiter mit der Mischung B in Reaktion zu bringen, qualitativ und quantitativ reproduzierbare Tetrapeptidkombinationen als Grundlage für beispielsweise ein therapeutisch wirksames Präparat zur Verfügung gestellt werden können. Bezüglich der gleichbleibenden Qualität und Quantität der Tetrapeptidkombinationen sei hier vorab auf die Figur 2 verwiesen, die den Vergleich von 3 unabhängig voneinander hergestellten Tetrapeptidkombinationen (GKL-02) in einer chromatographischen Auflösung (Chromatogramm) zeigt. Beispielweise können in dem von der Anmelderin hergestellten Präparat mit dem Namen GKL-02 die Molverhältnisse der Tetrapeptide, das heißt der Tetrapeptidbibliothek, die aus einer Mischung aus 11 Aminosäuren besteht, beispielsweise für die Aminosäure Asparaginsäure (Asp) 8,33 Mol-%, für die Aminosäure Glutaminsäure (Glu) 9,53 Mol-%, für die Aminosäure Prolin (Pro) 15, 18 Mol-% und für Glycin (Gly) 37,10 Mol-% usw. sein. Für die erfindungsgemäße Tetrapeptidkombination können sowohl natürliche als auch synthetische Aminosäuren oder eine Kombination aus natürlichen und synthetischen Aminosäuren eingesetzt werden.

Es ist vorgesehen, dass die Tetrapeptidkombination aus 11 Aminosäuren besteht, die mit 11 in der Aminosäuregruppe identischen Aminosäuren oder weiterführend ein aus 11 und 11 in ihrer Aminosäuregruppe identischen Aminosäuren hergestelltes Tetrapeptid mit 11 identischen Aminosäuren usw. kombinierbar ist.

Tetrapeptide bestehen aus vier Aminosäuren. Viele Tetrapeptide sind pharmakologisch aktiv und zeigen oft eine Affinität und Spezifität zu einer Vielzahl von Rezeptoren. Sowohl lineare als auch cyclische Tetrapeptide sind für die bevorzugten Tetrapeptide denkbar, wobei die cyclischen Tetrapeptide durch eine vierfache Peptidbindung oder durch kovalente Bindungen entstehen können. Beispiele für Tetrapeptide sind Tuftsin (L-threonyl-L-lysyl-L-prolyl-L-arginin), ein Gewebshormon der Phagozytose, Rigin (glycyl-L-glutaminyl-L-prolyl-L-arginin) mit ähnlichen Funktionen wie die des Tuftsin, Postin (Lys-Pro-Pro-Arg), welches das N-terminale Tetrapeptid des Cystatin C ist und ein Antagonist des Tuftsin ist, Endomorphin-1 (H-Tyr-Pro-Trp-Phe-NH₂) und Endomorphin-2 (H-Tyr-Pro-Phe-Phe-NH₂), Peptidamide mit der höchsten Affinität und Spezifität zu Opioidrezeptoren, die sich im zentralen und peripheren Nervensystem befinden und beispielsweise Tyrosin-MIF-1 (Tyr-Pro-Leu-Gly-NH₂), welches ein endogener Opioid- Modulator ist. Ganz allgemein sind die Peptidkombinationen im Bereich Onkologie, Immunologie, Endokrinologie und Dermatologie einsetzbar. Aber auch im Bereich Neurologie wie für die Endomorphine beschrieben und in anderen klinischen Bereichen sind die Peptidkombinationen einsetzbar.

Um die Aminosäuremischungen A und B miteinander in eine bestimmte Richtung zu einem Peptid synthetisieren zu können, müssen die Seitenkettenfunktionalitäten, wie bereits oben beschreiben, mittels Schutzgruppe geschützt und Schutzgruppen wieder abgespalten werden. Dazu werden je nach Synthese-Strategie geeignet geschützte Aminosäure-Bausteine eingesetzt. Folgende Schutzgruppen der Aminosäurefunktionalitäten können beispielsweise für die Synthese verwendet werden: Fluorenylmethyl-Oxy-Carbonyl (Fmoc) und tert-butyloxycarbonyl (Boc) für Aminogruppen, Benzylester (OBzl) und tert.-Butylester (OtBu) für Säuregruppen, 2,2,4,6,7-pentamethyldihydrobenzofuran-5-sulfonyl (Pbf), (2,2,5,7,8-pentamethyl-chroman-6-sulfonyl) Pmc, Trityl (Trt), Nitro (NO₂) als Schutz für die Seitenkettenfunktionalitäten. Vorzugsweise werden Boc-Aminosäuren mit Aminosäurebenzylester, Hydrochloriden oder -Tosylaten umgesetzt. Standard Kupplungsreagenzien wie z.B. N-Ethyl -N'-di-isopropyl-Carbodiimid (EDC, HCl), Dicyclohexylcarbodiimid (DCC), N ,N '-Di-isopropyl-carbodiimid (DIC) oder (2-(1H-Benzotriazole-1-yl)-I1,1,3,3-tetramethyluronium tetrafluoroborate) TBTU können für die Synthese der Peptide verwendet werden. Die Peptide werden stufenweise aufgebaut und am Ende der Synthese werden alle Schutzgruppen abgespalten.

Vorzugsweise wird die Peptidsynthese in Lösung durchgeführt, es ist aber auch eine Festphasensynthese denkbar. Bei der Synthese wird in einem ersten Schritt, wie bereits beschrieben, eine Mischung aus 11 Aminosäure-Benzylestern (Mischung A) mit einer entsprechenden Mischung aus 11 Boc-Aminosäuren (Mischung B) umgesetzt. Nach Abspaltung der Boc-Schutzgruppe wird in einem zweiten Schritt wieder mit der gleicher Mischung der 11 BocAminosäuren (Mischung B) reagiert. Nach Spaltung der Boc-Schutzgruppe bei der durch die oben beschriebenen Schritte entstandenen Tripeptid-Kombinationen wird wiederum mit einer Mischung der 11 Aminosäuren, deren Aminofunktionen mit der Benzyloxycarbonyl (Z)-Gruppe geschützt sind, umgesetzt. Die Z-Schutzgruppe wird am Ende durch Hydrierung mit Wasserstoff abgespalten. Nach Aufbau der Tetrapeptidbibliothek und Abspaltung aller Schutzgruppen wird das Zwischenprodukt lyophilisiert. Der erhaltenen entschützten Tetrapeptidbibliothek wird zur Stabilisierung und zur Erhöhung der Funktionalität bzw. Spezifität eine Mischung aus natürlichen Aminosäuren in folgendem Verhältnis von 100 g +/- 25 g Peptide : 80 g +/- 20 g Aminosäuremischung zugesetzt. Hierzu werden die Tetrapeptidkombinationen in Wasser gelöst und gegebenenfalls mit einer wässrigen Lösung der Aminosäuren H-Asp-OH 2,41 Mol-%; H-Thr-OH 9,26 Mol-%; H-Ser-OH 8,61 Mol-%; H-Glu-OH 6,44 Mol-%; H-Pro-OH 2,30 Mol-%; H-Gly-OH 4,94 Mol-%; H-Ala-OH 11,69 Mol-%; H-Cys-OH.HCl 0,47 Mol-%; H-Val-OH 6,76 Mol-%; H-Met-OH 3,24 Mol-%; H-Ile-OH 4,25 Mol-%; H-Leu-OH 14,15 Mol-%; H-Tyr-OH 0,36 Mol-%; H-Phe-OH 4,27 Mol-%; H-His-OH.HCl.H₂0 1,22 Mol-%; H-Lys-OH.HCl 10,36 Mol-% und H-Arg-OI-1.HCP 9,28 Mol-% versetzt und anschließend lyophilisiert. Natürlich ist je nach Anwendung der Tetrapeptidkombinationen in einem Präparat auch eine andere Mischung als die oben genannte Mischung der zugemischten Aminosäuren in einem frei wählbaren Molverhältnis denkbar.

Erfindungsgemäß ist ein Syntheseverfahren von Tetrapeptidkombinationen wobei Tetrapeptide aus einer Mischung (A), enthaltend
- eine Anzahl x an Aminosäuren mit geschützter Säuregruppe oder eine Anzahl z an Peptiden mit mittels Schutzgruppe geschützter Säuregruppe und aktivierter Aminogruppe, wobei die Aminosäuren in der Mischung (A) in bestimmten einstellbaren Molverhältnissen vorliegen,
   und einer Mischung (B), enthaltend
- eine Anzahl y an Aminosäuren mit mittels Schutzgruppe geschützter Aminogruppe, wobei die Aminosäure-Molverhältnisse der Mischung (B) gleich den Aminosäure-Molverhältnisse der Mischung (A) sind, und
wobei die Anzahl x = y ist, synthetisiert werden, und wobei Tetrapeptide aus Mischungen (A) und (B) enthaltend jeweils 11, 12, 13, 14, 15, 16, 17 oder 18 Aminosäuren synthetisiert werden.

Vorzugsweise dient das erfindungsgemäße Verfahren zur Synthese von Tetrapeptidkombinationen bzw. zur Erstellung einer Tetrapeptidbibliothek, so dass zur Vermeidung von Wiederholungen auf die Vorteilsangaben zu der erfindungsgemäßen Tetrapeptidkombination im vorigen Teil der Beschreibung und auf die nachfolgende Beschreibung zu der Tetrapeptidbibliothek verwiesen wird.

In bevorzugter erfindungsgemäßer Ausführung des Verfahrens wird jedoch noch einmal ausdrücklich auf den vorzugsweisen Zusatz einer Mischung aus natürlichen Aminosäuren in wässriger Lösung hingewiesen, wobei in einem vorangehenden Schritt nach Synthese der Tetrapeptide die Seitenkettenfunktionalitäten durch Abspaltung der Schutzgruppen aktiviert werden und in einem weiteren vorangehenden Zwischenschritt vor Versetzen der synthetisierten Tetrapeptide mit der Mischung aus natürlichen Aminosäuren in wässriger Lösung, die synthetisierten Peptide lyophilisiert werden und nach Versetzen mit der Mischung aus natürlichen Aminosäuren erneut lyophilisiert werden, um ein Pulver zu gewinnen.

Die vorliegende Erfindung wird durch die im Folgenden gezeigten und diskutierten Figuren sowie durch das Beispiel genauer erläutert.

### Beispiel

### 1. Synthese von Tetrapeptiden / Tetrapeptidbibliothek

### 1.1. Reagenzien, Lösungsmittel und zusätzliche Chemikalien

Hydroxybenzotriazol Monohydrat (HOBt * H₂0); O-(1H-Benzotriazol-1-yl)-I 1,3,3-tetramethyluronium tetrafluoroborat (TBTU); N,N-Diisopropylethylamin (DIPEA); Ethylacetat (EtOAc); N,N-Dimethylformamid (DMF); Toluen; Trifluoressigsäure (TFA); Eisessig (AcOH); Palladium (Pd), 10% ad Kohlenstoff, mit 50% Wasser versetzt; Destilliertes Wasser und Natriumhydrogencarbonat (NaHCO3).

### 2. Syntheseschritt 1: Boc-AA-OH + H-AA-OBzl • TOS oder • HCl -> Boc-AA-AA-OBzl

### 2.1. Ausgangsmaterialien

### Mischung B

- Boc-Ala-OH, Boc-Arg(N0₂)-OH, Boc-Asp(OBzl)-OH, Boc-Glu(OBzl)-OH, Boc-Gly-OH, Boc-Ile-OH • 1/2 H₂0, Boc-Leu-OH • H₂0, Boc-Lys(Z)-OH, Boc-Phe-OH, Boc-Pro-OH, Boc-Val-OH

### Mischung A

- H-Ala-OBzl • TOS, H-Arg(N0₂)-OBzl • TOS, H-Asp(OBzl)-OBzl • TOS, H-Glu(OBzl)-OBzl TOS H-Gly-OBzl • TOS, H-Ile-OBzl • TOS, H-Leu-OBzl • TOS, H-Lys(Z)-OBzl • HCl, Phe-OBzl • HCl, H-Pro-OBzl • HCl, H-Val-OBzl • TOS.

### 2.2. Syntheseverfahren

Ein Reaktorgefäß, beispielsweise ein 250 l mit Glas ausgekleidetes Gefäß, wird bis 0,6 bis 0,9 mbar Vakuum beaufschlagt, mit Stickstoff gereinigt und mit DMF (18,4 kg) beladen. Dann werden die folgenden Ausgangsmaterialien hinzugefügt: Boc-Asp(OBzl)OH (553,771g), Boc-Glu(OBzl)-OH (661,065 g), Boc-Pro-OH (671.664 g), Boc-Gly-OH (1335,986 g), Boc-Ala-OH (455,612 g), Boc-Val-OH (258,295 g), Boc-He-OH • 1/2 H₂0 (152,469 g), Boc-Lys(Z)-OH (376,679 g), Boc-Arg(N0₂)-OH (235,826 g), Boc-Leu-OH •. H₂0 (7,789 g), Boc-Phe-OH (38,337 g).

Ein zweites, beispielsweise ein 250 l mit Glas ausgekleidetes Gefäß, wird bis 0,6 bis 0,9 mbar Vakuum beaufschlagt mit Stickstoff gereinigt und mit DMF (19,4 kg) beladen. Die folgenden Aminosäurebenzylester-Tosylate bzw. Hydrochloride werden hinzugefügt:
H-Asp(OBzl)-OBzl • TOS (831,556 g), H-Glu(OBzl)-OBzl • TOS (978,910 g), H-Pro-OBzl • HCl (754,260 g), H-Gly-OBzl • TOS (2573,058 g), H-Ala-OBzl• TOS (846,208 g), H-Val-OBzl TOS (451,143 g), H-Ile-OBzl TOS (249,683 g), H-Lys(Z)OBzl • HCl (402,887 g), H-Arg(N02)-OBzl 2 TOS (483,12 g), H-Leu-OBzl • TOS (12,294 g), H-Phe-OBzl • HCl (42,162 g).

Der Inhalt des ersten Reaktors wird vollständig in den zweiten Reaktor überführt. Unter geringer Stickstoffbelüftung wird HOBt Monohydrat (3,78kg) bei 20 - 25°C hinzugegeben und die Mischung bei 4-6°C gerührt, bis eine vollständige Auflösung erreicht ist. Zu dieser Lösung wird TBTU (7,92kg) hinzugegeben, wobei eine Aufschwämmung erfolgt. Zu der Lösung wird DIPEA (9,996kg) hinzugegeben, während die Temperatur unter 15°C gehalten wird. Die Temperatur wird dann auf 25°C erhöht und die Mischung für ungefähr 15 bis 20 h gerührt, gefolgt von einer azeotropen Destillation mit Toluen (2 x 14 kg), um das gesamte DMF zu entfernen, gefolgt von der Entfernung des Toluens mit EtOAc (2 x 9,3 kg). Nach erneuter Zugabe von EtOAc (28 kg) wird die Reaktionsmischung mit einer 8%-igen NaHCO₃-Lösung (51 L) und dann mit Wasser (30 kg) extrahiert und unter Vakuum bis zum Erhalt eines öligen Rests aufkonzentriert, der im nächsten Syntheseschritt 2 verwendet wird.

### 3. Syntheseschritt 2: Boc-AA-AA-OBzl - H-AA-AA-OBzl • TFA

### 3.1. Ausgangsmaterialien

Der ölige Rest aus Syntheseschritt 1 und TFA

### 3.2. Syntheseverfahren

Ein Reaktorgefäß, beispielsweise ein 250 l mit Glas ausgekleidetes Gefäß, enthaltend den öligen Rest aus dem vorangegangen Schritt, wird bis 0,6 bis 0,9 mbar Vakuum beaufschlagt und nach Reinigung mit Stickstoff mit Trifluoressigsäure (TFA, 30,4 kg) innerhalb von 10 - 30 min beladen, wobei die Temperatur unter 30°C gehalten wird. Eine Aufkonzentration bei 45°C führt zu einem öligen Rest, zu dem Toluen (32,8 kg) hinzugegeben werden, um des restliche TFA durch azeotrope Destillation zu entfernen. Um die komplette Entfernung des TFA's sicher zu stellen, wird sukzessiv EtOAc (9,8 kg) und destilliertes Wasser (10,3 kg) hinzugegeben. Dann wird die Reaktionsmischung zu einem Öl aufkonzentriert und unter Vakuum bei 35 °C aufbewahrt. Das Öl wird in dem nachfolgenden Syntheseschritt verwendet.

### 4. Syntheseschritt 3: Boc-AA-OH + H-AA-AA-OBzl • TFA - Boc-AA-AA-AA-OBzl

### 4.1. Ausgangsmaterialien

Der ölige Rest aus Syntheseschritt 2 und Boc-Ala-OH Boc-Arg(N0₂)-OH Boc-Asp(OBzl)-OH Boc-Glu(OBzl)-OH, Boc-Gly-OH, Boc-Ile-OH • 1/2 H₂0, Boc-Leu-OH • H₂0, Boc-Lys(Z)-OH, Boc-Phe-OH, Boc-Pro-OH, Boc-Val-OH.

### 4.2. Syntheseverfahren

Ein Reaktorgefäß, beispielsweise ein 250 l mit Glas ausgekleidetes Gefäß, wird bis 0,6 bis 0,9 mbar Vakuum beaufschlagt, mit Stickstoff gereinigt und mit DMF (17,4 kg) beladen. Bei Raumtemperatur werden die folgenden Produkte hinzugegeben: BocAsp(OBzl)-OH (553,771 g), Boc-Glu(OBzl)-OH (661,065 g), Boc-Pro-OH (671,664 g), BocGly-OH (1335,986 g), Boc-Ala-OH (455,612 g), Boc-Val-OH (258.295 g), Boc-Ile-OH 1/2 H₂0 (152,469 g), Boc-Lys(Z)-OH (376,679 g), Boc-Arg(N0₂)-OH (235,826 g), Boc-Leu-OH • H₂0 (7,789 g), Boc-Phe-OH (38,337 g).

Ein zweites mit Glas ausgekleidetes Gefäß, enthaltend den öligen Rest aus dem vorangegangenen Schritt, wird mit Vakuum beaufschlagt und mit Stickstoff gereinigt. Dann wird DMF (17,4 kg) hinzugegeben und die Mischung bis zum Erhalt einer homogenen Lösung gerührt. Der Inhalt des ersten Reaktors wird vollständig in den zweiten Reaktor überführt und HOBt Monohydrat (3,78 kg) hinzugegeben und die gesamte Mischung bis zur kompletten Auflösung aller Inhaltsstoffe gerührt. An diesem Punkt (komplette Auflösung) wird die Lösung auf 5°C heruntergekühlt und HBTU (7,92 kg) hinzugegeben, gefolgt von der Zugabe von DIPEA (9,996 kg), während die Temperatur unter 10°C gehalten wird. Diese Lösung wird bei etwa 55°C unter Vakuum bis zum Erhalt eines öligen Rests aufkonzentriert, der einer azeotropen Destillation als Folge der Zugabe von Toluen, EtOAc, und Wasser unterzogen wird und dann mittels Vakuums weiter aufkonzentriert wird. Zu dem erhaltenen öligen Rest wird EtOAc hinzugegeben und die organische Lösung mehrmals mit 8%-iger Natriumhydrogencarbonat-Lösung (NaHCO₃), gefolgt von Wasser extrahiert und dann evaporiert, um einen öligen Rest zu erhalten, der im nächsten Schritt verwendet wird.

### 5. Syntheseschritt 4: Boc-AA-AA-AA-OBzl - H-AA-AA-AA-OBzl • TFA

### 5.1. Ausgangsmaterialien

Der ölige Rest aus Syntheseschritt 3 und TFA.

### 5.2. Syntheseverfahren

Ein Reaktorgefäß, beispielsweise ein 250 l mit Glas ausgekleidetes Gefäß, enthaltend den öligen Rest aus dem vorangegangen Schritt, wird bis 0,6 bis 0,9 mbar Vakuum beaufschlagt und nach Reinigung mit Stickstoff mit Trifluoressigsäure (TFA, 30,4 kg) innerhalb von 10 - 30 min bei einer Temperatur von etwa 25 - 30°C beladen. Durch Evaporation bei 45°C wird ein öliger Rest erhalten, zu dem Toluen (32,8 kg) hinzugegeben und die Mischung aufkonzentriert wird. Um die komplette Entfernung von TFA sicherzustellen wird EtOAc (9,8 kg) und destilliertes Wasser (10,3 kg) sukzessiv hinzugegeben. Das Reaktionsgemisch wird zu einem Öl aufkonzentriert und unter Vakuum bei 35°C aufbewahrt. Dieses Gemisch wird für den folgenden Schritt 5 verwendet.

### 6. Syntheseschritt 5: Z-AA-OH + H-AA-AA-AA-OBzl • TFA - Z-AA-AA-AA-AA-OBzl

### 6.1. Ausgangsmaterialien

Z-Ala-OH, Z-Arg(N02)-OH, Z-Asp(OBzl)-OH, Z-Glu(OBzl)-OH, Z-Gly-OH, Z-Ile-OH, Z-Leu-OH, Z-Lys(Z)-OH, Z-Phe-OH, 1-Pro-OH, Z-Val-OH und der ölige Rest aus Syntheseschritt 4: H-AA-AA-AA-OBzl • TFA.

### 6.2. Syntheseverfahren

Ein Reaktorgefäß, beispielsweise ein 250 l mit Glas ausgekleidetes Gefäß wird unter Stickstoff-Atmosphäre mit DMF (17,4 kg) beladen und bei Raumtemperatur gerührt. N_{α}-Carboxybenzyl geschützte Aminosäuren (1-AA-OH) werden wie folgt hinzugegeben:
Z-Asp(OBzl)-OH (612.017 g), Z-Glu(OBzl)-OH (727.726 g), Z-Pro-OH (777.819 g), Z-Gly-OH (1 595,434 g), Z-Ala-OH (537,531 g), Z-Val-OH (298,741 g), Z-Ile-OH (168,344 g), Z-Lys(Z)OH (410,362 g), Z-Arg(N0₂)-OH (260,943 g), Z-Leu-OH (8,289 g) and Z-Phe-OH (43,253 g). Ein zweites, beispielsweise ein 250 l mit Glas ausgekleidetes Gefäß, wird bis 0,6 bis 0,9 mbar Vakuum beaufschlagt und mit Stickstoff gereinigt. Dieses Gefäß wird mit dem öligen Rest aus Schritt 4, nämlich mit H-AA-AA-AAOBzl • TFA und DMF (19.4 kg) beladen und die Mischung bis zum Erhalt einer homogenen Lösung gerührt. Der Inhalt des ersten Gefäßes (Reaktors) wird vollständig in den zweiten Reaktor überführt.

Bei Raumtemperatur wird HOBt Monohydrat (3,78kg) hinzugegeben und die Mischung, bis das gesamte HOBt Monohydrate aufgelöst ist. Unter geringer Stickstoffbelüftung wird TBTU (7,92 kg) und DIPEA (9,996 kg) langsam (60 - 90 min) bei einer Temperatur von etwa 4-6°C hinzugegeben und der pH-Wert wiederholt gemessen, um sicherzustellen, dass dieser innerhalb eines Wertebereichs von 6,5 bis 7,0 pH bleibt.

Die Aufkonzentrierung dieser Lösung bei etwa 55°C unter Vakuum ergibt einen öligen Rest, der einer azeotropen Destillation durch Zugabe und Aufkonzentrierung nach jedem Schritt mit den folgenden Lösungsmitteln unterzogen wird: Toluen, EtOAc / Wasser. Zu dem öligen Rest, der unter Evaporation der letzten Lösung erhalten wird, wird EtOAc hinzugegeben und die Lösung mehrmals mit 8%-iger Natriumhydrogencarbonat-Lösung (NaHCO₃) und Wasser extrahiert. Die zusammen geführte organische Phase wird zu einem Öl aufkonzentriert, welches im folgenden Schritt 6 verwendet wird.

### 7. Syntheseschritt 6: Z-AA-AA-AA-AA-OBzl H-AA-AA-AA-AA-OH

### 7.1. Ausgangsmaterialien

Restöl aus Schritt 5: Z-AA-AA-AA-AA-OBzl.

### 7.2. Syntheseverfahren

Zu dem öligen Rest aus dem vorangegangenen Schritt 5 wird AcOH (86,3 kg) hinzugegeben und die Mischung bei etwa 25°C gerührt, bis eine komplette Auflösung erfolgt ist. Anschließend wird diese Lösung hydriert.

Dazu wird ein Hydrierungsreaktor (beispielsweise 630 L) mit einem Pd-Katalysator (10% ad C, 50% H₂O) beladen und durch Schmelzen inaktiviert. Die Tetrapeptid-Bibliothek Z-AA-AA-AA-AA-OBzl in AcOH wird vollständig in den Reaktor, enthaltend den Pd-Katalysator, überführt. Wasserstoffgas wird eingeleitet und die Hydrierung erfolgt bei einer Maximaltemperatur von 40°C und bei 2500 bis 3000 mbar (absoluter Druck).

Nach Beendigung der Reaktion wird das Reaktionsgemisch durch Filtrierung aufgereinigt und das reine Filtrat zu einem Öl evaporiert, welches dann durch sukzessive Zugabe von großen Mengen EtOAc aufgearbeitet und evaporiert wird. Das erhaltene Öl wird in Wasser gelöst und die Lösung (etwa 8 - 12 %) wird im nächsten Schritt 7 lyophilisiert.

### 8. Syntheseschritt 7: Lyophilisation der Tetrapeptide H-AA-AA-AA-AA-OH

### 8.1. Ausgangsmaterialien

Wässrige Lösung aus Schritt 6: H-AA-AA-AA-AA-OH in H₂O.

### 8.2. Syntheseverfahren

Die Lyophilisation erfolgt wie folgt:
- Sublimierung: Die Lösung wird auf -40°C innerhalb von 3 h gekühlt. Dann erfolgt die Erhöhung der Temperatur von -40°C auf 50°C unter Vakuum bei 200 µbar innerhalb von 48 h;
- Desorption: Die Temperatur wird bei 50°C unter 20 µbar während 24 h aufrechterhalten.

### 9. Syntheseschritt 8: H-AA-AA-AA-AA-OH + H-AA-OH -> H-AA-AA-AA-AA-OH, H- AA-OH

### 9.1. Ausgangsmaterialien

H-Asp-OH, H-Thr-OH, H-Ser-OH, H-Glu-OH, H-Pro-OH, H-Gly-OH, H-Ala-OH, H-Cys • HCl, H-Val-OH, H-Met-OH, H-Ile-OH, H-Leu-OH, H-Tyr-OH, H-Phe-OH, H-His-OH • H20 • HCl, HLys-OH • HCl, H-Arg-OH • HCl.

### 9.2. Syntheseverfahren

In ein mit Wasser beladenes und mit Stickstoff gereinigtes Glas ausgekleidetes Gefäß (beispielsweise 250 L) wird das lyophilisierte Tetrapeptid (A g) aus dem vorangegangenen Schritt und die folgenden L-Aminosäuren hinzugegeben: H-Asp-OH (1,559 g x (f)), H-Thr-OH (5,364g x (f)), H-Ser-OH (4,402g x (f)), H-Glu-OH (4,610g x (f)), H-Pro-OH (1,285g x (f)), H-Gly-OH (1,803g x (f)), H-Ala-OH (5,066g x (f)), HCys • HCl (0361g x (f)), H-Val-OH (3,848g x (f)), H-Met-OH (2,348g x (f)), H-Ile-OH (2,711g x (f)), H-Leu-OH (9,028g x (f)), H-Tyr-OH (0,317g x (f)), H-Phe-OH (3,429g x (f)), H-His-OH, H₂0 • HCl (1,242g x (f)), H-Lys-OH • HCl (9,210g x (f)), H-Arg-OH • HCl (9,512g x (f)), wobei (f) = A g lyophilisierte Tetrapeptid-Bibliothek_{found} /g Peptid-Bibliothek_{norm batch} ist.

Bei einer Temperatur von etwa 40°C wird die erhaltene Lösung gerührt, bis eine komplette Auflösung erfolgt ist. Dann wird die Temperatur auf 20 - 25°C erniedrigt und die Lösung über Nacht gerührt. Anschließend erfolgt die Filtrierung der Peptid-Lösung, um ein reines Filtrat zu erhalten, dass im folgenden Schritt 9 lyophilisiert wird.

### 10. Syntheseschritt 9: Lyophilisation von GKL02; Pharmakon Substanz

### 10.1. Ausgangsmaterialien

### Das Filtrat aus Schritt 8

### 10.2. Syntheseverfahren

Die Lyophilisation erfolgt wie folgt:
- Sublimierung: Die Lösung wird auf -40°C innerhalb von 3 h gekühlt. Dann erfolgt die Erhöhung der Temperatur von -40°C auf 50°C unter Vakuum bei 200 µbar innerhalb von 48 h;
- Desorption: Die Temperatur wird bei 50°C unter 20 µbar während 24 h aufrechterhalten.

Die erhaltene Substanz aus Schritt 9, die als medizinisch wirksames Präparat einsetzbar ist, kann nachfolgend in Pulverform, zu Tabletten gepresst, in Kapseln gefüllt oder in sonstiger generischer Form abgepackt und bei einer Temperatur unter - 15°C gelagert werden.

### Figuren

Die Figuren zeigen:
- Fig. 1: ein Flussdiagramm des in Beispiel 1 beschriebenen Syntheseverfahrens und
- Fig. 2: zeigt Chromatogramme von aufeinander folgenden Chargen von GKL-02 mit internem Standard.

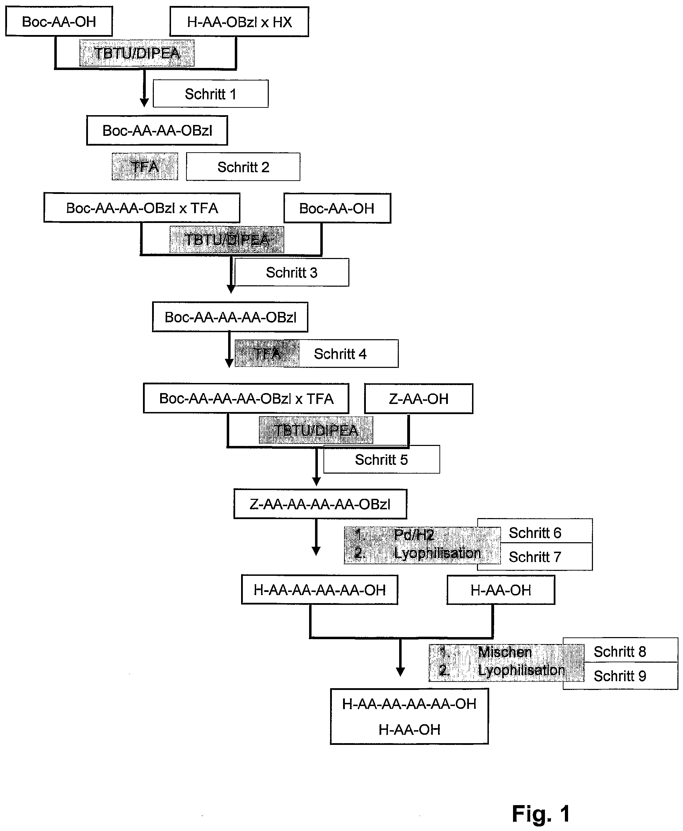

Figur 1 zeigt ein Flussdiagramm eines Syntheseverfahrens für eine erfindungsgemäße Tetrapeptidbibliothek bzw. für erfindungsgemäße Tetrapeptide. Zur Erläuterung des Flussdiagramms wird zur Vermeidung von Wiederholungen auf die obige Beschreibung zu Beispiel 1 verwiesen.

Die Figur 2 zeigt Chromatogramme von aufeinander folgenden Chargen von GKL-02: Charge 1=GKL02 SC02808L1; Charge 2=GKL02 15935-AA/2 und Charge 3=GKL02 15507-AA/9. Anhand von internen Standards (siehe Pfeile) sind bei allen drei Chargen indentische Muster der aufeinanderfolgenden, identischen Peaks ermittelbar, was Rückschlüsse auf die hervorragende Konsistenz, dass heißt auf die Reproduzierbarkeit der Tetrapeptid-Bibliotheken, die mit dem erfindungsgemäßen Verfahren hergestellt werden, zulässt. Insbesondere sind die gezeigten Chromatogramme ein Beweis für die konsistente reproduzierbare Qualität und Quantität der erfindungsgemäßen Peptidkombinationen.

## Patentansprüche

1. Tetrapeptidkombination, wobei die Aminosäuren der Tetrapeptidkombination aus einer Anzahl x von verschiedenen Aminosäuren bestehen, wobei x = 11 ist, und wobei die Tetrapeptidkombination hergestellt wird durch:
a) Reaktion einer ersten Mischung (A), umfassend die Anzahl x verschiedener Aminosäuren mit einer geschützten Säuregruppe, wobei die Aminosäuren in der ersten Mischung in bestimmten einstellbaren Molverhältnissen vorliegen, mit
einer ersten Mischung (B), umfassend die Anzahl x von Aminosäuren mit Aminogruppen, die durch Schutzgruppen geschützt sind, wobei die Aminosäure-Molverhältnisse in der ersten Mischung (B) gleich den Aminosäure-Molverhältnissen in der ersten Mischung (A) sind;
b) Aktivieren der geschützten Aminogruppen der resultierenden Dipeptidkombination, durch Spaltung der Schutzgruppen, wodurch eine Dipeptidkombination erzeugt wird, die Dipeptide mit geschützten Säuregruppen und aktivierten Aminogruppen umfasst;
c) Reaktion einer zweiten Mischung (A), umfassend die Dipeptidkombination, die Dipeptide mit geschützten Säuregruppen und aktivierten Aminogruppen umfasst; mit
einer zweiten Mischung (B), die die Anzahl x von Aminosäuren mit Aminogruppen umfasst, die durch Schutzgruppen geschützt sind, wobei die Aminosäure-Molverhältnisse in der zweiten Mischung (B) gleich den Aminosäure-Molverhältnissen in der zweiten Mischung (A) sind;
d) Aktivieren der geschützten Aminogruppen der resultierenden Tripeptidkombination, durch Spaltung der Schutzgruppen, wodurch eine Tripeptidkombination erzeugt wird, die Tripeptide mit geschützten Säuregruppen und aktivierten Aminogruppen umfassen;
e) Reaktion einer dritten Mischung (A), umfassend die Tripeptidkombination, die Tripeptide mit geschützten Säuregruppen und aktivierten Aminogruppen umfasst; mit
einer dritten Mischung (B), umfassend die Anzahl x von Aminosäuren mit Aminogruppen, die durch Schutzgruppen geschützt sind, wobei die Aminosäure-Molverhältnisse in der dritten Mischung (B) gleich den Aminosäure-Molverhältnissen in der dritten Mischung (A) sind.

2. Tetrapeptidkombination nach Anspruch 1, **dadurch gekennzeichnet, dass** den Peptiden eine Mischung aus natürlichen Aminosäuren in einem Verhältnis von 100 g +/- 25 g Peptide: 80 g +/- 20 g Aminosäuremischung zugesetzt ist.

3. Verfahren zur Synthese von Tetrapeptidkombinationen, wobei das Verfahren die Schritte umfasst:
a) Reaktion einer ersten Mischung (A), umfassend die Anzahl x verschiedener Aminosäuren mit einer geschützten Säuregruppe, wobei die Aminosäuren in der ersten Mischung in bestimmten einstellbaren Molverhältnissen vorliegen, mit
einer ersten Mischung (B), umfassend die Anzahl x von Aminosäuren mit Aminogruppen, die durch Schutzgruppen geschützt sind, wobei die Aminosäure-Molverhältnisse in der ersten Mischung (B) gleich den Aminosäure-Molverhältnissen in der ersten Mischung (A) sind;
b) Aktivieren der geschützten Aminogruppen der resultierenden Dipeptidkombination, durch Spaltung der Schutzgruppen, wodurch eine Dipeptidkombination erzeugt wird, die Dipeptide mit geschützten Säuregruppen und aktivierten Aminogruppen umfasst;
c) Reaktion einer zweiten Mischung (A), umfassend die Dipeptidkombination, die Dipeptide mit geschützten Säuregruppen und aktivierten Aminogruppen umfasst; mit
einer zweiten Mischung (B), die die Anzahl x von Aminosäuren mit Aminogruppen umfasst, die durch Schutzgruppen geschützt sind, wobei die Aminosäure-Molverhältnisse in der zweiten Mischung (B) gleich den Aminosäure-Molverhältnissen in der zweiten Mischung (A) sind;
d) Aktivieren der geschützten Aminogruppen der resultierenden Tripeptidkombination, durch Spaltung der Schutzgruppen, wodurch eine Tripeptidkombination erzeugt wird, die Tripeptide mit geschützten Säuregruppen und aktivierten Aminogruppen umfassen;
e) Reaktion einer dritten Mischung (A), umfassend die Tripeptidkombination, die Tripeptide mit geschützten Säuregruppen und aktivierten Aminogruppen umfasst; mit
einer dritten Mischung (B), umfassend die Anzahl x von Aminosäuren mit Aminogruppen, die durch Schutzgruppen geschützt sind, wobei die Aminosäure-Molverhältnisse in der dritten Mischung (B) gleich den Aminosäure-Molverhältnissen in der dritten Mischung (A) sind, und wobei x= 11, 12, 13, 14, 15, 16, 17 oder 18 ist.

4. Verfahren nach Anspruch 3, **dadurch gekennzeichnet, dass** Tetrapeptide aus Peptiden mit einer Sequenzlänge (SEQL) ≥ 2 und einer Mischung (B) synthetisiert werden, wobei die Peptide an ihrer Säuregruppe geschützt sind und an ihrer Aminogruppe aktiviert werden.

5. Verfahren nach Anspruch 3 oder 4, **dadurch gekennzeichnet, dass** die 11, 12, 13, 14, 15, 16, 17 oder 18 Aminosäuren in einer Mischung (A) in einem Molverhältnis eingesetzt werden, welches dem der Aminosäuren der Mischung (B) gleich ist.

6. Verfahren nach einem der Ansprüche 3 bis 5, **dadurch gekennzeichnet, dass** die Aminosäuren oder die Peptide mit SEQL ≥ 2 der Mischung (A), die zur weiteren Synthese mit der Mischung (B) eingesetzt werden, und die Aminosäuren der Mischung (B) an ihren Seitenkettenfunktionalitäten mittels Schutzgruppe geschützt sind.

7. Verfahren nach Anspruch 6, **dadurch gekennzeichnet, dass** nach Synthese der Tetrapeptide die geschützten Säure-Aminogruppen und Seitenkettenfunktionalitäten durch Abspaltung der Schutzgruppen aktiviert werden, und dass in einem Zwischenschritt die Peptide lyophilisiert werden und in wässriger Lösung mit einer Mischung aus natürlichen Aminosäuren vermischt werden, wobei das Verhältnis der Peptide zu der Aminosäuremischung 100 g +/- 25 g Peptide : 80 g +/- 20 g Aminosäuremischung ist, und/oder in einem folgenden Schritt die Peptid-Aminosäuremischung lyophilisiert wird.

8. Tetrapeptid-Bibliothek, bestehend aus einer Tetrapeptidkombination gemäß Anspruch 1.

9. Therapeutisches Präparat enthaltend eine Tetrapetidkombination nach Anspruch 1 oder 2.

10. Tetrapeptidkombinationen nach Anspruch 1 oder 2 zur Verwendung in der Onkologie, Immunologie, Endokrinologie oder Dermatologie.

## Claims

1. Tetrapeptide combination, wherein the amino acids of the tetrapeptide combination comprise a number x of different amino acids, wherein x = 11, and wherein the tetrapeptide combination is produced by means of:
a) reacting a first admixture (A) comprising the number x of different amino acids with a protected acid group, wherein the amino acids are present in the first admixture in specific adjustable molar ratios, with
a first admixture (B) comprising the number x of amino acids with amino groups which are protected by protection groups, wherein the amino acid molar ratios in the first admixture (B) are equal to the amino acid molar ratios in the first admixture (A);
b) activating the protected amino groups of the resulting dipeptide combination by splitting the protection groups, whereby a dipeptide combination which comprises dipeptides with protected acid groups and activated amino groups is produced;
c) reacting a second admixture (A) comprising the dipeptide combination which comprises dipeptides with protected acid groups and activated amino groups; with
a second admixture (B) which comprises the number x of amino acids with amino groups which are protected by protection groups, wherein the amino acid molar ratios in the second admixture (B) are equal to the amino acid molar ratios in the second admixture (A);
d) activating the protected amino groups of the resulting tripeptide combination by splitting the protection groups, whereby a tripeptide combination which comprises tripeptides with protected acid groups and activated amino groups is produced;
e) reacting a third admixture (A) comprising the tripeptide combination which comprises tripeptides with protected acid groups and activated amino groups; with
a third admixture (B) comprising the number x of amino acids with amino groups which are protected by protection groups, wherein the amino acid molar ratios in the third admixture (B) are equal to the amino acid molar ratios in the third admixture (A).

2. Tetrapeptide combination according to claim 1, **characterised in that** an admixture of natural amino acids in a ratio of 100 g ± 25 g peptides: 80 g ± 20 g amino acid admixture is added to the peptides.

3. Method for synthesis of tetrapeptide combinations, wherein the method comprises the steps of:
a) reacting a first admixture (A) comprising the number x of different amino acids with a protected acid group, wherein the amino acids are present in the first admixture in specific adjustable molar ratios, with
a first admixture (B) comprising the number x of amino acids with amino groups which are protected by protection groups, wherein the amino acid molar ratios in the first mixture (B) are equal to the amino acid molar ratios in the first admixture (A);
b) activating the protected amino groups of the resulting dipeptide combination by splitting the protection groups, whereby a dipeptide combination which comprises dipeptides with protected acid groups and activated amino groups is produced;
c) reacting a second admixture (A) comprising the dipeptide combination which comprises dipeptides with protected acid groups and activated amino groups; with
a second admixture (B) which comprises the number x of amino acids with amino groups which are protected by protection groups, wherein the amino acid molar ratios in the second admixture (B) are equal to the amino acid molar ratios in the second admixture (A);
d) activating the protected amino groups of the resulting tripeptide combination by splitting the protection groups, whereby a tripeptide combination which comprises tripeptides with protected acid groups and activated amino groups is produced;
e) reacting a third admixture (A) comprising the tripeptide combination which comprises tripeptides with protected acid groups and activated amino groups; with
a third admixture (B) comprising the number x of amino acids with amino groups which are protected by protection groups, wherein the amino acid molar ratios in the third admixture (B) are equal to the amino acid molar ratios in the third admixture (A), and wherein x = 11, 12, 13, 14, 15, 16, 17 or 18.

4. Method according to claim 3, **characterised in that** tetrapeptides comprising peptides with a sequence length (SEQL) ≥ 2 and an admixture (B) are synthesised, wherein the peptides are protected at the acid group thereof and are activated at the amino group thereof.

5. Method according to claim 3 or 4, **characterised in that** the 11, 12, 13, 14, 15, 16, 17 or 18 amino acids in an admixture (A) are used in a molar ratio which is equal to that of the amino acids of the admixture (B).

6. Method according to any one of claims 3 to 5, **characterised in that** the amino acids or the peptides with SEQL ≥ 2 of the admixture (A) which are used for further synthesis with the admixture (B) and the amino acids of the admixture (B) are protected at the side chain functionalities thereof by means of a protection group.

7. Method according to claim 6, **characterised in that**, after synthesis of the tetrapeptides, the protected acid amino groups and side chain functionalities are activated by splitting the protection groups, and **in that** in an intermediate step the peptides are lyophilised and mixed in aqueous solution with an admixture of natural amino acids, wherein the ratio of peptides to the amino acid admixture is 100 g ± 25 g of peptides: 80 g ± 20 g of amino acid admixture and/or in a following step the peptide/amino acid admixture is lyophilised.

8. Tetrapeptide library comprising a tetrapeptide combination according to claim 1.

9. Therapeutic preparation containing a tetrapeptide combination according to claim 1 or 2.

10. Tetrapeptide combinations according to claim 1 or 2 for use in oncology, immunology, endocrinology or dermatology.

## Revendications

1. Combinaison de tétrapeptides, les acides aminés de la combinaison de tétrapeptides étant constitués d'un nombre x de différents acides aminés, avec x = 11, la combinaison de tétrapeptides étant produite par :
a) réaction d'un premier mélange (A), comprenant le nombre x de différents acides aminés présentant un groupe acide protégé, les acides aminés étant présents dans le premier mélange selon certains rapports molaires pouvant être ajustés, avec
un premier mélange (B), comprenant le nombre x d'acides aminés présentant des groupes amino protégés par des groupes protecteurs, les rapports molaires des acides aminés du premier mélange (B) étant les mêmes que les rapports molaires des acides aminés dans le premier mélange (A) ;
b) activation des groupes amino protégés de la combinaison de dipeptides obtenue, par dissociation des groupes protecteurs, avec en conséquence production d'une combinaison de dipeptides qui comprend des dipeptides présentant des groupes acide protégés et des groupes amino activés ;
c) réaction d'un second mélange (A), comprenant la combinaison de dipeptides, qui comprend des dipeptides présentant des groupes acide protégés et des groupes amino activés ; avec
un second mélange (B), qui comprend le nombre x d'acides aminés présentant des groupes amino, protégés par des groupes protecteurs, les rapports molaires des acides aminés du second mélange (B) étant les mêmes que les rapports molaires des acides aminés du second mélange (A) ;
d) activation des groupes amino protégés de la combinaison de tripeptides obtenue, par dissociation des groupes protecteurs, avec production d'une combinaison de tripeptides qui comprennent des tripeptides présentant des groupes acide protégés et des groupes amino activés ;
e) réaction d'un troisième mélange (A) comprenant la combinaison de tripeptides, qui comprend des tripeptides présentant des groupes acide protégés et des groupes amino activés ; avec
un troisième mélange (B) comprenant le nombre x d'acides aminés présentant des groupes amino, protégés par des groupes protecteurs, les rapports molaires des acides aminés du troisième mélange (B) étant les mêmes que les rapports molaires des acides aminés du troisième mélange (A).

2. Combinaison de tétrapeptides selon la revendication 1, **caractérisée en ce qu'**on ajoute aux peptides un mélange d'acides aminés naturels dans un rapport de 100 g +/- 25 g de peptides:80 g +/- 20 g du mélange d'acides aminés.

3. Procédé de synthèse de combinaisons de tétrapeptides, le procédé comprenant les étapes suivantes :
a) réaction d'un premier mélange (A), comprenant le nombre x de différents acides aminés présentant un groupe acide protégé, les acides aminés étant présents dans le premier mélange selon certains rapports molaires pouvant être ajustés, avec
un premier mélange (B), comprenant le nombre x d'acides aminés présentant des groupes amino protégés par des groupes protecteurs, les rapports molaires des acides aminés du premier mélange (B) étant les mêmes que les rapports molaires des acides aminés dans le premier mélange (A) ;
b) activation des groupes amino protégés de la combinaison de dipeptides obtenue, par dissociation des groupes protecteurs, avec en conséquence production d'une combinaison de dipeptides qui comprend des dipeptides présentant des groupes acide protégés et des groupes amino activés ;
c) réaction d'un second mélange (A), comprenant la combinaison de dipeptides, qui comprend des dipeptides présentant des groupes acide protégés et des groupes amino activés ; avec
un second mélange (B), qui comprend le nombre x d'acides aminés présentant des groupes amino, protégés par des groupes protecteurs, les rapports molaires des acides aminés du second mélange (B) étant les mêmes que les rapports molaires des acides aminés du second mélange (A) ;
d) activation des groupes amino protégés de la combinaison de tripeptides obtenue, par dissociation des groupes protecteurs, avec production d'une combinaison de tripeptides qui comprennent des tripeptides présentant des groupes acide protégés et des groupes amino activés ;
e) réaction d'un troisième mélange (A) comprenant la combinaison de tripeptides, qui comprend des tripeptides présentant des groupes acide protégés et des groupes amino activés ; avec
un troisième mélange (B) comprenant le nombre x d'acides aminés présentant des groupes amino, protégés par des groupes protecteurs, les rapports molaires des acides aminés du troisième mélange (B) étant les mêmes que les rapports molaires des acides aminés du troisième mélange (A), et dans lequel x = 11, 12, 13, 14, 15, 16, 17 ou 18.

4. Procédé selon la revendication 3, **caractérisé en ce que** les tétrapeptides sont synthétisés à partir de peptides présentant une longueur de séquence (SEQL) ≥ 2 et d'un mélange (B), les peptides étant protégés au niveau de leur groupe acide et étant activés au niveau de leur groupe amino.

5. Procédé selon la revendication 3 ou 4, **caractérisé en ce que** les 11, 12, 13, 14, 15, 16, 17 ou 18 acides aminés se trouvant dans un mélange (A) sont utilisés selon un rapport molaire qui est le même que celui des acides aminés du mélange (B) .

6. Procédé selon l'une des revendications 3 à 5, **caractérisé en ce que** les acides aminés ou les peptides ayant une SEQL ≥ 2 du mélange (A), qui sont utilisés pour une synthèse ultérieure avec le mélange (B), et les acides aminés du mélange (B), sont sur leurs fonctionnalités en chaîne latérale protégés par un groupe protecteur.

7. Procédé selon la revendication 6, **caractérisé en ce que**, après la synthèse des tétrapeptides, les groupes acide-amino protégés et les fonctionnalités en chaîne latérale sont activés par dissociation des groupes protecteurs, et **en ce que**, dans une étape intermédiaire, les peptides sont lyophilisés et mélangés en solution aqueuse à un mélange d'acides aminés naturels, le rapport des peptides au mélange d'acides aminés étant de 100 g +/- 25 g de peptides : 80 g +/-20 g du mélange d'acides aminés, et/ou, dans une étape suivante, le mélange peptides-acides aminés est lyophilisé.

8. Bibliothèque de tétrapeptides consistant en une combinaison de tétrapeptides selon la revendication 1.

9. Préparation thérapeutique contenant une combinaison de tétrapeptides selon la revendication 1 ou 2.

10. Combinaisons de tétrapeptides selon la revendication 1 ou 2 pour une utilisation en oncologie, immunologie, endocrinologie ou dermatologie.
